# EUROPEAN PATENT APPLICATION

(11) **EP 3 482 693 A1**
(43) Date of publication of application: **15.05.2019**
(21) Application number: 17200712.2
(22) Date of filing: 09.11.2017
(51) Int. Cl.: A61B 10/00, B01L 3/00

(54) **BIOPSY CONTAINER WITH IDENTIFIER**

(71) Applicant: Koninklijke Philips N.V., 5656 AE Eindhoven (NL)
(72) Inventor: VAN DER ZAAG, Pieter Jan, 5656 AE Eindhoven (NL); VAREKAMP, Christiaan, 5656 AE Eindhoven (NL)
(74) Representative: de Haan, Poul Erik

(57) **Abstract**

The invention relates to a biopsy container (10) with an identification mark (13), which comprises one or more curves that surround the biopsy container. Identification information of the biopsy container may be encoded in a plurality of widths of the curves surrounding the biopsy container as well as in distances between these curves. Additionally, the biopsy container may comprise an alignment mark (11), which is configured to facilitate the registration of images of the biopsy container. The invention also relates to systems configured to determine identification information of a biopsy container and an image processing method.

## Description

### FIELD OF THE INVENTION

The invention relates to a biopsy container with an identification mark, a system configured to detect the identification mark of a biopsy container, and a method for processing images of a biopsy container.

### BACKGROUND OF THE INVENTION

Biopsies are commonly performed for determining the presence or extent of a disease such as a tumor. In a biopsy, tissue is extracted by a physician, and the tissue is analyzed subsequently by a pathologist. Depending on the type of disease to be detected, dozens of biopsies may need to be taken. Conventionally, for each biopsy, a physician navigates a biopsy needle to the location-of-interest inside the body, extracts the tissue sample and places it in a dedicated vessel for further processing. In order to keep a trace for each biopsy, the vessel is identified by a specific label on the vessel including identification information such as the patient's name, the location from which the sample was taken etc.

European patent application EP 3217887 A1 relates to a biopsy device for taking a three-dimensional (3D) biopsy comprising an outer sleeve, a hollow main shaft, a biopsy tube and a tube shaft. The hollow main shaft may have a distal end portion with a sideward facing notch, and the main shaft may be adapted to be accommodated within the outer sleeve. A proximal end of the biopsy tube may be attachable to a distal end of the tube shaft, so that it is movable together with the tube shaft within the hollow main shaft between a proximal position, in which the biopsy tube is not located in the notch, and a distal position, in which the biopsy tube is located in the notch.

International patent application WO 2017/042650 A2 relates to a system for planning and performing interventional procedures. The system includes a registration device and an image generation device, which map current targets in a reference image for a first interventional procedure to at least one guidance image acquired from a different imaging modality. Biopsy locations are recorded to the guidance images during the first interventional procedure and the biopsy locations are mapped to the reference image to provide a planning image for use in a subsequent interventional procedure on the patient. In a subsequent interventional procedure, the prior planning image may be registered to a current reference image and the prior biopsy locations and prior and current targets are mapped to a guidance image acquired from a different imaging modality. Biopsy locations are then mapped to the guidance image and mapped back to the current reference image.

International patent application WO 2017/109201 A1 relates to the processing of a 3D tissue sample, comprising the steps of receiving a tube with an inner space and two open ends, wherein the tube is configured to retain the 3D tissue sample in the inner space, arranging the tube so that one of the two open ends of the tube is located at a fluid channel, and forcing or actively pressing a tissue processing agent through the fluid channel and into the tube so that the tissue processing agent passes through the tissue.

### SUMMARY OF THE INVENTION

There may be a need to provide a more reliable and efficient way to manage multiple biopsies. For example, for the detection and analysis of prostate cancer, up to around 50 biopsies may be taken. The biopsies may be taken simultaneously or sequentially from different locations under the guidance of an imaging system. For the proper analysis of the extracted tissue and for defining an appropriate treatment, it may be important to know the location and orientation of each biopsy container at the time when the tissue sample is extracted. This location and orientation information may be used, for example, to determine accurately the position and/or size of a tumor.

The object of the present invention may be seen as to reduce the complexity of performing multiple biopsies. In particular, it is an object of the present invention to make the process of performing multiple biopsies less error-prone and less time consuming. The locations of an organ from where tissue samples are extracted by means of biopsies are to be made available so that the analysis results of the tissue samples can be associated accurately and readily to certain locations of the organ.

The problem of the present invention is solved by the subject-matter of the independent claims, wherein further embodiments are incorporated in the dependent claims.

The described embodiments similarly pertain to the biopsy container, the system and the method for processing medical images. Synergetic effects may arise from different combinations of the embodiments although they might not be described in detail.

Furthermore, it shall be noted that all embodiments of the present invention concerning a method might be carried out with the order of the steps as described. Nevertheless, this has not to be the only and essential order of the steps of the method. The methods presented herein can be carried out with another order of the disclosed steps without departing from the respective method embodiment, unless explicitly mentioned to the contrary hereinafter.

Technical terms are used by their common sense. If a specific meaning is conveyed to certain terms, definitions of terms will be given in the following in the context of which the terms are used.

According to the present invention, a biopsy container is presented. The biopsy container comprises an identification mark for identifying the biopsy container, wherein the identification mark comprises a curve, which surrounds the interior of the biopsy container. The biopsy container may preferably have the shape of a tube with at least one open end. The interior of the biopsy container may have for example a circular cross-section orthogonal to a longitudinal axis of the biopsy container. Furthermore, the biopsy container itself may have a circular circumference in a cross-section orthogonal to the longitudinal axis of the biopsy container. The curve of the identification mark may lie within a plane orthogonal to the longitudinal axis of the biopsy container. Preferably, the curve surrounding the interior of the biopsy container is a closed curve.

The curve of the identification mark may be formed on the outer surface of the biopsy container for example by modifying the structure of the outer surface or by modifying the material close to the outer surface. Alternatively, the curve of the identification mark may be formed on the inner surface of the biopsy container for example by modifying the structure of the inner surface or by modifying the material close to the inner surface. Alternatively, the curve may be embedded into the material of the biopsy container.

The identification mark is configured to identify the biopsy container. In particular, the identification mark may be configured to identify multiple biopsy containers used for one patient. However, the identification mark may also be configured to identify larger numbers of biopsy containers, for example to identify biopsy containers used for several patients.

Preferably, a physician utilizes a medical imaging system such as an ultrasound or magnetic resonance imaging system to obtain image information of the region-of-interest of the patient during the process of taking a biopsy. In particular, the medical imaging system may be utilized to navigate a biopsy container towards the location-of-interest inside the body from where tissue is to be extracted. Furthermore or alternatively, the medical imaging system may be utilized to map biopsies to locations inside the body from where tissue samples have been extracted. The identification mark of the biopsy container may be configured such that it is detectable by the medical imaging system so that images provided by the medical imaging system allow to determine identification information of the biopsy container. For example, if the medical imaging system is a magnetic resonance imaging system, superparamagnetic iron oxide nanoparticles may be embedded in the identification mark to facilitate the detection of the identification mark in images generated by the magnetic resonance imaging system. Furthermore, the identification mark of the biopsy container is preferably configured such that it is detectable by means of an imaging unit of a biopsy scanner, so that images provided by the imaging unit of the biopsy scanner allow to determine identification information of the biopsy container.

The focal plane of an imaging unit may cut the curve of the identification mark, which surrounds the interior of the biopsy container, at two points. An image generated by the imaging unit may be processed to determine these two points by searching for in-focus regions in the image. The two crossing points, where the curve intersects with the focal plane of the imaging unit, may be used to determine identification information of the biopsy container.

In an exemplary embodiment, the curve spans an entire circumference of the biopsy container. In other words, the curve may surround the biopsy container. Thereby, the curve may lie on an outer surface of the biopsy container. For example, the curve may be printed on a label, which may be glued onto the outer surface of the biopsy container such that it fully surrounds the biopsy container. Alternatively, the curve may be formed by modifying the outer surface of the biopsy container or by modifying the material close to the outer surface of the biopsy container.

In another exemplary embodiment, identification information is encoded in the thickness of the curve surrounding the interior of the biopsy container. For example, curves with thicknesses/widths of 10, 15, 20 or 30 µm may be utilized to identify different biopsy containers. In another exemplary embodiment, identification information is encoded in the color of the curve surrounding the interior of the biopsy container. For example, red, green and blue curves may be utilized to identify different biopsy containers. In another exemplary embodiment, identification information is encoded in the transparency of the curve surrounding the interior of the biopsy container. For example, curves with different levels of transparency for certain imaging modalities may be utilized to identify different biopsy containers.

In another exemplary embodiment, the identification mark comprises at least two curves, which surround the interior of the biopsy container, and the identification information is encoded in distances between the at least two curves surrounding the interior of the biopsy container. The at least two curves, which surround the interior of the biopsy container, preferably lie within planes, which are essentially orthogonal to the longitudinal axis of the biopsy container. Distances between the two curves of for example 20, 40 and 60 µm may be utilized to identify different biopsy containers.

The focal plane of an imaging unit may cut each of the at least two curves surrounding the interior of the biopsy container at two points. These two crossing points (per curve) may be determined by searching for in-focus regions of an image of the biopsy container. For each curve surrounding the interior of the biopsy container, a line segment, which connects the two crossing points, may be determined. Then, distances between these line segments of different curves surrounding the interior of the biopsy container may be determined. Identification information of the biopsy containers may be determined subsequently by decoding the distances between the line segments that connect the two in-focus points of each curve surrounding the interior of the biopsy container. Herein, a line is an unbounded straight curve. In addition, a line segment is a straight curve of finite length.

The at least two curves may at least partially obstruct the tissue sample within the biopsy container so that a biopsy scanner may not be able to provide information about obstructed parts of the tissue sample. To avoid substantial obstructions of the tissue sample by the identification mark, the curves surrounding the interior of the biopsy container are preferably manufactured such that regions between those curves remain transparent. Hence, the identification mark may be configured such that a biopsy scanner can gather information about those parts of the tissue sample inside the biopsy container, which are located between curves of the identification mark.

In another exemplary embodiment, the biopsy container comprises an alignment mark for registering images of the biopsy container. Images of the biopsy container may be generated for example by an imaging unit of a medical imaging system such as an ultrasound or magnetic resonance imaging system used to obtain image information of the region-of interest of the patient during the process of taking a biopsy. Furthermore, images of the biopsy container may be generated by the imaging unit of a biopsy scanner. The alignment mark is preferably configured so that it can be detected on images generated by the medical imaging system as well as on images generated by the imaging unit of the biopsy scanner. The alignment mark on the biopsy container is configured to facilitate the registration of images of the biopsy container.

In particular, the alignment mark may be configured such that image data provided by the biopsy scanner can be correlated with image data provided by the medical imaging system used to obtain image information of the region-of-interest of the patient during the process of taking a biopsy. The biopsy scanner may generate a 3D analysis of the tissue sample inside the biopsy container. The image data provided by the medical imaging system may represent an organ of the patient. As a result, the 3D analysis of the tissue sample generated by the biopsy scanner may be correlated with a certain location within the organ depicted on the image generated by the medical imaging system. This may facilitate, for example, the accurate assessment of the location and growth of a tumor.

The alignment mark of the biopsy container may serve to correlate images of the biopsy container generated with several imaging modalities such as magnetic resonance imaging, X-ray, ultrasound, etc. Furthermore, the alignment mark of the biopsy container may serve to correlate images taken from different imaging directions. This may result in a 3D analysis of the tissue sample.

The alignment mark may be small and/or sufficiently thin not to occlude important features in the images. The alignment mark may be straightforward to recognize in an image stack, and/or may allow unambiguous determination of 3D location and orientation of the biopsy container relative to the imaging unit.

A microscopic z-stack or tilted angle z-stack comprises image data for multiple focal planes. By means of the alignment mark, these images may be registered into a single coordinate system. This applies also for the case of a plurality of z-stack scans, each corresponding to a different scanning direction of the biopsy container. Then, images of the plurality of z-stack scans may be registered in a common coordinate system based on an analysis of the locations and orientations of the alignment mark in the images.

Above requirements for the functional form of the alignment mark may lead to an alignment mark comprising a line segment arranged in parallel to a longitudinal axis of the biopsy container. Such a line segment is preferable as it does not occlude large parts of the tissue sample inside the biopsy container, is straightforward to recognize in an image stack, and may be utilized for high precision estimation of 3D location and orientation of the biopsy container. Of course, a single line segment does not uniquely define a coordinate system. To reduce ambiguities, the alignment mark may comprise a second line segment, which may be arranged in parallel to the first line segment of the alignment mark. Ambiguities may also be eliminated by indicating starting points of the line segments using suitable starting symbols. Each image in a focal z-stack may have only part of a line segment in focus. A calibration procedure may start by first finding an image that has a starting symbol in focus. Then, images of the z-stack can be analyzed sequentially to track the crossing points of the two line segments of the alignment mark with the focal planes of the z-stack through the sequence of images by searching for in-focus points within the images. In each image, local searches may be performed for the pixel that is sharpest and satisfies some darkness criterion. After determining the crossing points of the two line segments with the focal planes of the z-stack, line equations in the image acquisition coordinate system can be determined. Subsequently, image analysis results can be mapped into a coordinate system of the biopsy container.

The alignment mark is preferably configured to uniquely define a coordinate system so that detecting the alignment mark facilitates the registration of image data of the biopsy container for arbitrary imaging directions. The alignment mark may comprise a line segment, which may be continuous or interrupted, such as a dotted or dashed line segment or combinations thereof. In an example, the alignment mark comprises a curve. Exemplarily, the curve is continuous, as e.g. a spiral. Exemplarily, the curve is interrupted, as e.g. a dotted or dashed spiral or combinations thereof. In an example, the alignment mark comprises two line segments or curves. The second line segment or curve may eliminate ambiguities for the determination of a coordinate system based on the alignment mark. In an example, the two line segments or curves are arranged in parallel to each other. In another example, the two line segments or curves are arranged at different parts of the biopsy container. Exemplarily, the two line segments or curves are arranged at different ends of the biopsy container. Exemplarily, the alignment mark comprises three line segments or curves. Exemplarily, the alignment mark comprises a combination of at least a line segment and a curve.

The alignment mark may be attached to the biopsy container in the same manufacturing step as the identification mark. Hence, the same technology may be utilized to form the alignment and identification marks of the biopsy container resulting in a cost and time efficient production of the biopsy container comprising these marks.

The alignment and identification marks may be configured such that both marks can be detected in images provided by the imaging unit of a biopsy scanner and, optionally, by the imaging unit of a medical imaging system used to obtain image information of the region-of-interest of the patient during the process of taking a biopsy. For example, if the medical imaging system is a magnetic resonance imaging system, superparamagnetic iron oxide nanoparticles may be embedded in the identification and alignment marks to facilitate the detection of these marks in images generated by the magnetic resonance imaging system. Preferably, neither the medical imaging system nor the biopsy scanner requires a separate imaging unit for acquiring the identification and alignment marks of the biopsy container.

The alignment mark may also be utilized to determine the location and orientation of the biopsy container inside the biopsy device of a biopsy gun. The biopsy device is used for taking biopsies and may be configured so that its location and orientation can be determined from images generated by the medical imaging system. Then, the location and orientation of the biopsy container may be derived from the location and orientation of the biopsy device on the image generated by the medical imaging system and the location and orientation of the biopsy container inside the biopsy device determined based on the alignment mark.

The biopsy container may comprise a container orientation mark to indicate a specific rotational orientation of the biopsy container relative to a tube shaft of a biopsy device, such that the biopsy container can only be inserted into the biopsy device in a single, predefined way. For example, the container orientation mark may be a visual mark or a mechanic mark. In an example, one of the container orientation mark and the shaft orientation mark comprises a protrusion (as e.g. an edge) and the other an indention (as e.g. a groove) matching the protrusion. The protrusion or indention forming the mechanic container orientation mark may visually indicate a specific rotational orientation of the biopsy container relative to the tube shaft.

In another exemplary embodiment, the identification mark and the alignment mark are at least partially integrated into a joint identification and alignment mark. As noted above, an alignment mark may comprise one or more curves. A joint identification and alignment mark may for example comprise a dashed curve, wherein the identification information is encoded in lengths of dashes of the dashed curve and/or in lengths of gaps between dashes of the dashed curve. Alternatively, the joint identification and alignment mark may for example comprise a curve, which is modulated with a spatial frequency to encode identification information. Hence, biopsy containers may be identified by detecting the spatial frequency of the curve. Alternatively, the joint identification and alignment mark may for example comprise a spiral, wherein identification information is encoded in a pitch of the turns of the spiral. In this alternative, different lengths of the pitch may correspond to different identifiers of the biopsy container.

In another exemplary embodiment, the alignment mark and the identification mark are attached to the biopsy container before taking a biopsy. Preferably, the alignment and identification marks are attached to the biopsy container before taking a biopsy such that these marks can be detected in images generated during the process of taking the biopsy. This facilitates the correlation of image data generated during the process of taking the biopsy with, for instance, image data generated by a biopsy scanner when analyzing the tissue sample within the biopsy container.

In another exemplary embodiment, the alignment mark or the identification mark are attached to the biopsy container at one end of the biopsy container or at opposing ends of the biopsy container. Attaching the identification mark at an end of the biopsy container may be preferable to reduce obstructions of the tissue sample in the biopsy container caused by the identification mark. Similarly, attaching the alignment mark at an end of the biopsy container may be preferable to reduce obstructions of the tissue sample in the biopsy container caused by the alignment mark.

According to the present invention, also a system is presented. The system comprises an imaging unit configured for generating an image. Furthermore, the system comprises a processing unit configured for detecting the identification mark of a biopsy container in said image and configured for determining identification information of the biopsy container based on the detected identification mark. For example, the system may be a biopsy gun, which is configured to detect the identification mark of the biopsy container that will be utilized for the next biopsy. Alternatively, the system may be a medical imaging system used to obtain image information of the region-of-interest of the patient during the process of taking a biopsy. The imaging unit and the processing unit of such a medical imaging system may serve to detect and track the biopsy container that is currently in use. Furthermore or alternatively, the medical imaging system may be utilized to map biopsies to locations inside the body from where tissue samples have been extracted.

Alternatively, the system may be a biopsy scanner. The imaging unit and the processing unit of the biopsy scanner may be configured to generate a 3D analysis of the tissue sample inside the biopsy container based on a plurality of 2D images of the biopsy container. Additionally, the imaging unit and the processing unit of the biopsy scanner may detect the identification mark of the biopsy container and determine identification information of the biopsy container based on the detected identification mark. The identification information may be stored in association with the 3D analysis of the tissue sample.

Alternatively, the system may be a code reader configured to detect the identification mark of the biopsy container and to determine identification information of the biopsy container based on the detected identification mark. The code reader may be configured to store or output the identification information.

In an exemplary embodiment, the processing unit is configured for detecting the alignment mark of the biopsy container and for registering the image of the biopsy container based on the detected alignment mark. For example, the processing unit of a medical imaging system such as a magnetic resonance imaging system used to obtain imaging information of the region-of-interest of the patient during the process of taking a biopsy may be configured for detecting the alignment mark of the biopsy container. Towards this end, superparamagnetic iron oxide nanoparticles could be embedded in the alignment mark to facilitate the detection of the alignment mark in magnetic resonance images. Furthermore, the processing unit of a biopsy scanner may be configured for detecting the alignment mark of the biopsy container. The alignment mark is preferably configured so that it can be detected on images generated by the imaging unit of the medical imaging system as well as on images generated by the imaging unit of the biopsy scanner. The alignment mark on the biopsy container is configured to enable or support the registration of images of the biopsy container.

In particular, the alignment mark may allow to correlate image data provided by the biopsy scanner with image data provided by the medical imaging system used for obtaining image information of the region-of-interest of the patient during the process of taking a biopsy. The processing unit of the biopsy scanner may compute a 3D analysis of the tissue sample inside the biopsy container. The image data provided by the medical imaging system may represent an organ of the patient. As a result, the 3D analysis of the tissue sample generated by the biopsy scanner may be correlated with a certain location within the organ depicted on the image generated by the medical imaging system. This may facilitate, for example, the accurate assessment of the location and growth of a tumor.

The alignment mark of the biopsy container may serve to correlate images of the biopsy container generated using different imaging modalities such as magnetic resonance imaging, X-ray, ultrasound, etc. Furthermore, the alignment mark of the biopsy container may configured such that images of the biopsy container taken from different imaging directions can be registered. This may result in a 3D analysis of the tissue sample inside the biopsy container.

In another exemplary embodiment, the system is a biopsy gun. The biopsy gun may comprise a biopsy device for taking a biopsy. The biopsy device may comprise one or more biopsy containers. The imaging unit of the biopsy gun may generate an image of biopsy containers within the biopsy device. The processing unit of the biopsy gun may be configured to detect the identification marks of one or more biopsy containers based on the image generated by the imaging unit. The processing unit may also be configured to determine identification information of the detected identification marks of biopsy containers. Preferably, the imaging and processing units of the biopsy gun are configured to determine identification information of biopsy containers that will be used for the next biopsy.

The biopsy gun may also comprise an output unit for outputting identification information of the biopsy container and an input unit configured for receiving a trigger. Upon receiving the trigger, the input unit causes the biopsy device to take a biopsy and the output unit to output identification information of the biopsy containers that are utilized in the current biopsy.

The output unit of the biopsy gun may be connected to a receiving unit of a medical imaging system such as an ultrasound or magnetic resonance imaging system. The medical imaging system may be utilized by a clinician to obtain image information of the region-of-interest of the patient during the process of taking a biopsy. The medical imaging system may be adapted to generate an image upon receiving the identification information of one or more biopsy containers provided by the output unit of the biopsy gun. The input unit of the biopsy gun may be configured to cause the biopsy device to take the biopsy and the output unit to output the identification information of the currently used biopsy container(s) in an essentially synchronous manner. As a result, the medical imaging system connected with the output unit of the biopsy gun generates a new image at the time when the biopsy is taken. This image may be stored or output by the medical imaging system together with the identification information received from the output unit of the biopsy gun.

In other words, the system may be a medical imaging system for providing imaging information during the process of taking a biopsy. Preferably, the medical imaging system comprises a receiving unit for receiving identification information of a biopsy container. Upon receiving the identification information, the receiving unit causes the imaging unit of the medical imaging system to generate the image. The medical imaging system may store the generated image in a data structure together with the received identification information. The medical imaging system may for example be an ultrasound system or magnetic resonance imaging system. The receiving unit of the medical imaging system may be connected to an output unit of a biopsy gun. The output unit of the biopsy gun may be configured to output the identification information of one or more biopsy containers that are currently used for a biopsy. The transmission of identification information of one or more biopsy containers from the output unit of the biopsy gun to the receiving unit of the medical imaging system may trigger the imaging unit of the medical imaging system to generate an image. The generated image may be stored or output together with the identification information of the biopsy containers received from the output unit of the biopsy gun.

Alternatively, the output unit of the biopsy gun may send a trigger to the receiving unit of the medical imaging system. Upon receiving the trigger, the receiving unit of the medical imaging system causes the imaging unit of the medical imaging system to generate an image. Subsequently, the processing unit of the medical imaging system may detect the identification marks of one or more biopsy containers within the generated image. Furthermore, the processing unit may determine identification information of the detected identification marks of the biopsy containers. The medical imaging system may be configured to store or output the generated image together with the determined identification information of one or more biopsy containers.

Alternatively, the output unit of the biopsy gun may be configured to send identification information of the biopsy containers, which are used in the current biopsy, to a storage computer such as a remote server. Furthermore, the output unit of the biopsy gun may be configured to send a trigger to the receiving unit of the medical imaging system, which may be configured to cause, upon receiving the trigger, the imaging unit of the medical imaging system to generate an image. The medical imaging system may be configured to send the generated image to the storage computer, which may be configured to store the image received from the medical imaging system together with the identification information of biopsy containers received from the output unit of the biopsy gun.

In another exemplary embodiment, the system is a biopsy scanner for analyzing the sample inside the biopsy container. The processing unit of the biopsy scanner may be configured for generating an analysis, potentially a 3D analysis, of the tissue sample inside the biopsy container based on 2D images generated by the imaging unit of the biopsy scanner. The imaging unit of the biopsy scanner may be adapted to generate a z-stack of images of the biopsy container. In other words, the imaging unit of the biopsy scanner may be adapted to generate a sequence of images of the biopsy container for different focal planes. Furthermore, the imaging unit of the biopsy scanner may be adapted to generate images of the biopsy container from a plurality of imaging directions. The processing unit of the biopsy scanner may be configured to detect the alignment mark of the biopsy container within the images generated by the imaging unit of the biopsy scanner. Furthermore, the processing unit of the biopsy scanner may be configured to register image data of the tissue sample and to generate the 3D analysis.

According to the present invention, also a method for processing medical images is presented. The method comprises detecting the identification mark of a biopsy container according to the present invention and determining identification information of the identification mark of the biopsy container. The method is preferably executed by the processing unit of a system as defined above. In particular, the system may be a medical imaging system used for obtaining image information of the region-of-interest of the patient during the process of taking a biopsy. Alternatively, the method may be executed by the processing unit of a biopsy gun or by the processing unit of a biopsy scanner used for analyzing the tissue sample inside the biopsy container.

In an exemplary embodiment, the determination of identification information comprises determining the thickness of the curve surrounding the interior of the biopsy container and/or determining the distance between two curves surrounding the interior of the biopsy container.

In another exemplary embodiment, the determination of identification information comprises decoding a joint identification and alignment mark.

In another exemplary embodiment, the method further comprises detecting an alignment mark of the biopsy container in the images and performing a registration of the images by means of the alignment mark.

In another exemplary embodiment, the method further comprises detecting in-focus regions of the images and using the detected in-focus regions of the images for the detection of the biopsy container and for the determination of identification information.

In another exemplary embodiment, the method further comprises using the detected in-focus regions of the images for the detection of the alignment mark of the biopsy container and for the registration of the images.

According to the present invention, also a computer program element is presented, wherein the computer program element comprises program code means for causing the system as defined in the independent claim to carry out the steps of the image processing method as defined in the independent claim when the computer program is run on a computer controlling the imaging system.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the accompanying drawings:
Figure 1 shows schematically and exemplarily an embodiment of a biopsy container according to the invention.
Figure 2 shows schematically and exemplarily in-focus regions of identification and alignment marks in an image of a biopsy container according to the invention.
Figure 3 shows schematically and exemplarily another embodiment of a biopsy container according to the invention.
Figure 4 shows schematically and exemplarily another embodiment of a biopsy container according to the invention.
Figure 5 shows schematically and exemplarily systems configured for the detection of identification marks of biopsy containers according to the invention.
Figure 6 shows a schematic overview of steps of an image processing method according to the invention.

### DETAILED DESCRIPTION OF EMBODIMENTS

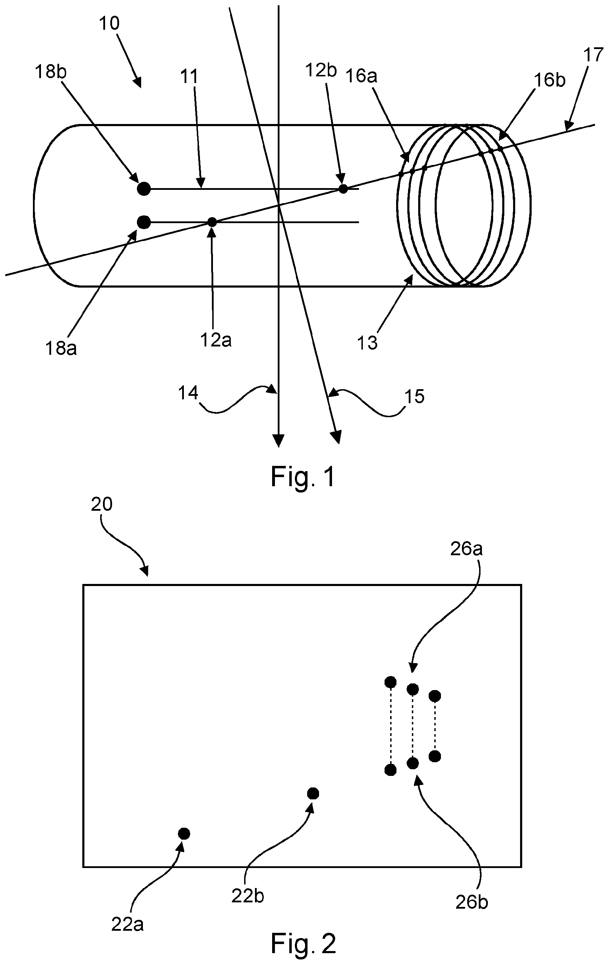

**Figure 1** shows schematically and exemplarily an embodiment of a biopsy container 10 according to the invention. The biopsy container 10 has the shape of a tube with two open ends. The biopsy container 10 has an identification mark 13, which comprises three circular curves surrounding the biopsy container. These three curves may lie within planes, which are orthogonal to the longitudinal axis of the biopsy container. In other examples, these curves may lie within planes, which make an angle different from 90° relative to the longitudinal axis of the biopsy container. Identification information may be encoded in varying widths of these curves surrounding the biopsy container and/or in varying distances between the curves surrounding the biopsy container. The biopsy container 10 also comprises an alignment mark 11, which includes two opaque, thin, line segments arranged in parallel to the longitudinal axis of the biopsy container. The alignment mark 11 also comprises two starting symbols 18a and 18b to indicate starting points of the line segments.

In Fig. 1, the z-axis 14 is defined orthogonal to the longitudinal axis of the biopsy container 10. The imaging direction 15 is inclined relative to the z-axis 14. For this reason, the focal plane 17 is also inclined relative to the longitudinal axis of the biopsy container. The focal plane 17 cuts the alignment mark 11 of the biopsy container 10 at crossing points 12a and 12b. Furthermore, the focal plane 17 cuts the identification mark at a triplet of points 16a and another triplet of points 16b.

For each imaging direction 15, an imaging unit may generate a z-stack of images, which is a sequence of images with focal planes 17 shifted in the direction of the z-axis 14. These images may show crossing points 12a and 12b of the focal planes with the alignment mark 11 of the biopsy container 10 at different image pixels. Analyzing the locations of the crossing points 12a and 12b in the sequence of z-stack images may allow to determine the location and orientation of the alignment mark. Subsequently, the location and orientation of the biopsy container 10 can be derived from the location and orientation of the alignment mark 11. At the same time, a single image may be sufficient to detect the identification mark 13 and to determine identification information of the biopsy container 10.

**Figure 2** shows schematically and exemplarily in-focus regions of identification and alignment marks in an image 20 of a biopsy container according to the present invention. The image depicted in Fig. 2 may be obtained for a biopsy container similar to the one shown in Fig. 1. In Fig. 2, out-of-focus image regions may have been suppressed. Furthermore, all image pixels with a brightness that exceeds a certain darkness threshold may have been suppressed, leaving only dark image pixels caused by opaque identification and alignment marks. Figure 2 illustrates crossing points 22a and 22b of the alignment mark with the focal plane of the imaging system. In addition, Fig. 2 shows two triplets 26a and 26b of crossing points of the focal plane with the identification mark. These triplets correspond to the three curves of the identification mark 13 surrounding the biopsy container 10 depicted in Fig. 1. In this example, a search for two triplets of nearby points may be performed to detect the identification mark of the biopsy container. For each curve of the identification mark, the two crossing points with the focal plane may be associated, which is illustrated by dashed line segments in Fig. 2. Identification information of the biopsy container may be determined by evaluating distances between these dashed line segments.

Figure 2 corresponds to an identification mark comprising three thin curves, resulting in crossing points with the focal plane of the imaging unit. In another example, the identification mark of the biopsy container may comprise curves of varying widths to encode identification information. In this example, the focal plane of the imaging unit may cut the identification mark at crossing strips of varying lengths. Identification information of the biopsy container may be determined by decoding the widths of these crossing strips.

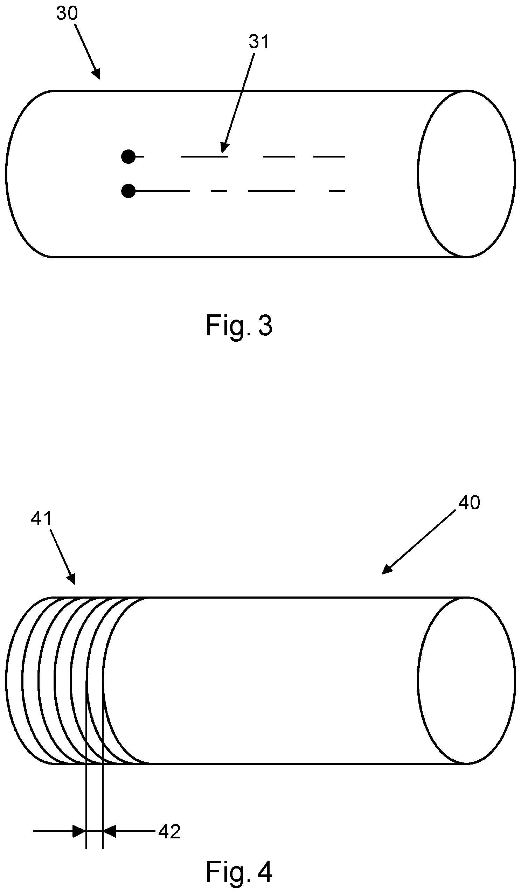

**Figure 3** shows schematically and exemplarily an embodiment of a biopsy container 30 according to the invention. The biopsy container 30 comprises a joint identification and alignment mark 31. The joint identification and alignment mark 31 comprises two dashed line segments. Identification information of the biopsy container may be encoded in a plurality of lengths of dashes and a plurality of lengths of gaps between dashes of the dashed line segments of the joint identification and alignment mark 31.

Gaps between dashes may cause that an image of the biopsy container does not show two crossing points of the focal plane with the joint identification and alignment mark. However, the alignment information may still be obtained from other images corresponding to other locations and/or orientations of the focal plane of the imaging unit (as the functional shape of the alignment mark may be known *a prior*).

**Figure 4** shows schematically and exemplarily an embodiment of a biopsy container 40 according to the invention. The biopsy container 40 comprises a joint identification and alignment mark 41. The joint identification and alignment mark 41 is a spiral surrounding the biopsy container 40, wherein identification information of the biopsy container is encoded in the pitch 42 between turns of the spiral.

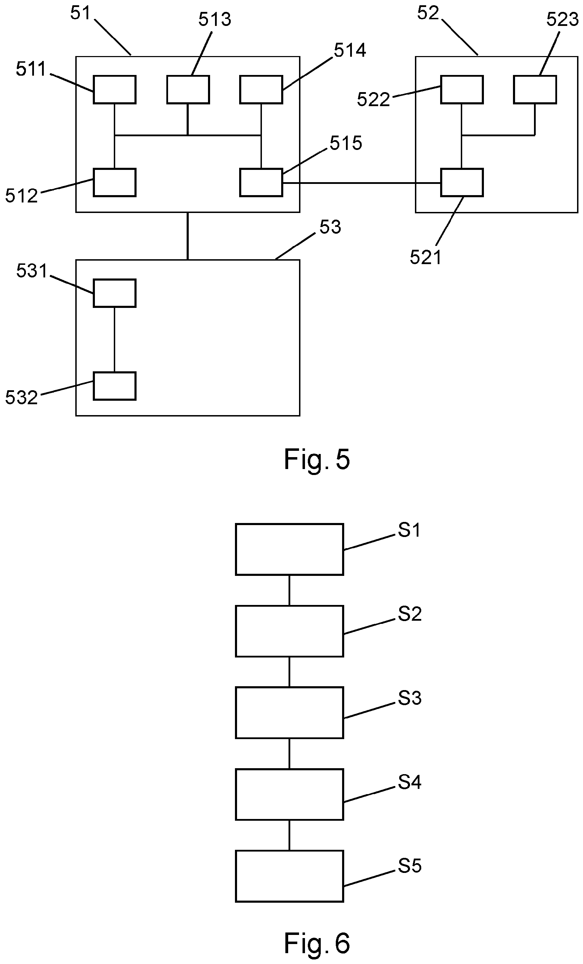

**Figure 5** shows schematically and exemplarily systems 51, 52 and 53 configured for the detection of identification marks of biopsy containers according to the invention. System 51 depicts a biopsy gun comprising a biopsy device 512 for taking a biopsy. The biopsy device 512 may comprise one or more biopsy containers. The biopsy gun 51 further comprises an imaging unit 513 for generating an image of biopsy containers of the biopsy device. The image generated by the imaging unit 513 is processed by the processing unit 514 to detect identification marks of biopsy containers and to determine identification information based on the detected identification marks. The biopsy gun 51 may also comprise an input unit 511 for receiving a trigger and an output unit 515 for outputting identification information of biopsy containers. The trigger may be a mechanical or electronic trigger. Upon receiving the trigger, the input unit 511 causes the biopsy device 512 to take a biopsy and the output unit 515 of the biopsy gun to output identification information of the biopsy containers that are used by the biopsy device 512 for the current biopsy.

The output unit 515 of the biopsy gun 51 may send the identification information of the biopsy containers to the receiving unit 521 of the medical imaging system 52. The medical imaging system 52 is used by a clinician, for example a radiologist, urologist or surgeon, to obtain image information of the region-of-interest inside the body of a patient during the process of taking a biopsy. Upon receiving identification information of biopsy containers from the output unit 515 of the biopsy gun 51, the receiving unit 521 causes the imaging unit 522 of the medical imaging system 52 to generate an image. Preferably, the imaging unit 522 generates an image of the part of the body that is to be examined by the biopsy. Furthermore, the imaging unit 522 preferably generates the image at the time when the biopsy is taken, such that the generated image shows the location from where the tissue sample is extracted. The medical imaging system 52 further comprises a processing unit 523 for processing the image generated by the imaging unit 522. The processing unit 523 may be configured to detect identification marks of one or more biopsy containers in the image generated by the imaging unit 522. Furthermore, the processing unit 523 of the medical imaging system may be configured to determine identification information of biopsy containers based on the detected identification marks. Thereby, the processing unit 523 may utilize the identification information of biopsy containers provided by the output unit 515 of the biopsy gun 51.

The processing unit 523 of the medical imaging system 52 may also be configured to process a plurality of images generated by the imaging unit 522 to detect alignment marks of one or more biopsy containers depicted in these images. Moreover, the processing unit 523 may be configured to derive the locations and orientations of one or more biopsy containers based on the detected alignment marks. Subsequently, the one or more images generated by the imaging unit 522 may be stored together with identification information of the biopsy containers and, potentially, location information of the biopsy containers.

In an alternative embodiment, the output unit 515 of the biopsy gun is configured to send identification information of the one or more biopsy containers that are used for the current biopsy to a storage computer, which may be a remote server. Furthermore, the output unit 515 may be configured to send a trigger to the receiving unit 521 of the medical imaging system 52. Upon receiving the trigger, the receiving unit 521 may cause the imaging unit 522 to generate one or more images of the patient. The processing unit 523 may process the images generated by the imaging unit 522 to determine identification and location information of the one or more biopsy containers. The medical imaging system 52 may send the one or more images generated by the imaging unit 522 and, potentially, the determined identification and location information of the one or more biopsy containers to the storage computer. This storage computer may store the image data in a data structure together with identification information of the biopsy containers and, potentially, location information of the biopsy containers.

Figure 5 also depicts a biopsy scanner 53 comprising an imaging unit 531 and a processing unit 532. The line between the biopsy gun 51 and the biopsy scanner 53 indicates that the biopsy scanner 53 receives a biopsy container with a tissue sample from the biopsy gun 51. The imaging unit 531 is configured to generate one or more images of the tissue sample inside the biopsy container. In particular, the imaging unit 531 of the biopsy scanner may generate z-stacks of images of the tissue sample inside the biopsy container for different imaging directions. The processing unit 532 may be adapted to process the images generated by the imaging unit 531. In particular, the processing unit 532 may be configured to detect the alignment mark of the biopsy container and to register the image data based on the detected alignment mark, potentially resulting in a 3D analysis of the sample tissue inside the biopsy container. In addition, the processing unit 532 may be configured to detect the identification mark of the biopsy container and to determine the associated identification information based on one or more images generated by the imaging unit 531. The biopsy scanner 53 may be configured to store or output the 3D analysis together with the determined identification information of the biopsy container.

**Figure 6** shows a schematic overview of steps of an image processing method 60 according to the invention. In step S1 of the image processing method 60, in-focus regions of one or more images are detected. Step S1 may also comprise the suppression of image structures in the one or more images, which are brighter than a darkness threshold. In step S2, the identification mark of a biopsy container according to the present invention is detected. In step S3, identification information of the detected identification mark is determined. In step S4, the alignment mark of the biopsy container is detected. In this step, a sequence of images may be analysed, and the crossing points of the alignment mark of the biopsy container with different focal planes of the imaging unit may be searched to determine the location and orientation of the alignment mark of the biopsy container. In step S5, image data is registered based on the detected alignment mark of the biopsy container. The registration of image data may be limited to image data from one imaging unit/imaging modality. Alternatively, the registration of image data may comprise image data from several imaging units/imaging modalities.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A biopsy container (10), comprising:
an identification mark (13) for identifying the biopsy container;
wherein the identification mark comprises a curve, which surrounds an interior of the biopsy container.

2. Biopsy container (10) according to claim 1,
wherein the curve spans an entire circumference of the biopsy container.

3. Biopsy container (10) according to any of claims 1 or 2,
wherein identification information is encoded in a thickness of the curve surrounding the interior of the biopsy container.

4. Biopsy container (10) according to any of claims 1 to 3,
wherein the identification mark (13) comprises at least two curves, which surround the interior of the biopsy container; and
wherein identification information is encoded in distances between the at least two curves surrounding the interior of the biopsy container.

5. Biopsy container (10) according to any of the preceding claims, further comprising:
an alignment mark (11) for registering images of the biopsy container.

6. Biopsy container (10) according to claim 5,
wherein the identification mark and the alignment mark are at least partially integrated into a joint identification and alignment mark (31).

7. Biopsy container (10) according to any of claims 5 or 6,
wherein the alignment mark (11) and the identification mark (13) are attached to the biopsy container before taking a biopsy.

8. Biopsy container (10) according to any of claim 5 to 7,
wherein the alignment mark (11) or the identification mark (13) are attached to the biopsy container at one end of the biopsy container or at opposing ends of the biopsy container.

9. A system (51, 52, 53), comprising:
an imaging unit (513, 522, 531) configured for generating an image of a biopsy container (10) according to any of the preceding claims; and
a processing unit (514, 523, 532) configured for detecting the identification mark (13) of the biopsy container in said image and configured for determining identification information of the biopsy container based on the detected identification mark.

10. System (51, 52, 53) according to claim 9,
wherein the processing unit (514, 523, 532) is configured for detecting the alignment mark (11) of the biopsy container (10); and
wherein the processing unit is configured for registering the image of the biopsy container based on the detected alignment mark.

11. System (51) according to any of claims 9 or 10,
wherein the system is a biopsy gun;
wherein the biopsy gun comprises a biopsy device (512) configured for taking a biopsy;
wherein the biopsy gun further comprises an output unit (515) for outputting identification information of the biopsy container (10);
wherein the biopsy gun further comprises an input unit (511) configured for receiving a trigger;
wherein the input unit is configured to cause, upon receiving the trigger, the biopsy device to take a biopsy and the output unit to output the identification information.

12. System (52) according to any of claims 9 or 10,
wherein the system is a medical imaging system for providing imaging information during a process of taking a biopsy,
wherein, preferably, the medical imaging system comprises a receiving unit (521) for receiving identification information from a biopsy gun according to claim 11;
wherein the receiving unit is configured to cause, upon receiving identification information, the imaging unit (522) to generate the image; and
wherein the medical imaging system is configured to store the image in a data structure together with the received identification information.

13. System (53) according to any of claims 9 or 10,
wherein the system is a biopsy scanner for analyzing a sample in the biopsy container (10);
wherein the processing unit (532) is configured for generating a three-dimensional analysis of the sample in the biopsy container based on images generated by the imaging unit (531).

14. Method (60) for processing medical images comprising the following steps:
detecting an identification mark (13) of a biopsy container (10) according to any of claims 1 to 8 in the images (S2);
determining identification information of the identification mark of the biopsy container (S3).

15. Method (60) according to claim 14,
wherein the determination of identification information comprises determining a thickness of the curve surrounding the interior of the biopsy container (10) and/or determining a distance between two curves surrounding the interior of the biopsy container.

16. Method (60) according to any of claims 14 or 15,
wherein the determination of identification information comprises decoding a joint identification and alignment mark (31).

17. Method (60) according to any of claims 14 to 16, further comprising:
detecting an alignment mark (11) of the biopsy container in the images (S4);
performing a registration of the images by means of the alignment mark (S5).

18. Method (60) according to any of claims 14 to 17, further comprising:
detecting in-focus regions of the images (S1);
using the detected in-focus regions of the images for the detection of the biopsy container (10) and for the determination of identification information.
